# EUROPEAN PATENT SPECIFICATION

(11) **EP 2 265 182 B1**
(45) Date of publication and mention of the grant of the patent: **07.05.2014**
(21) Application number: 08734676.3
(22) Date of filing: 19.03.2008
(51) Int. Cl.: A61B 8/12, A61M 25/10, A61M 25/09, A61M 25/01

(54) **SYSTEM FOR ULTRASONIC IMAGING OF AN ORGAN IN A PATIENT'S BODY THROUGH A PART OF THE PATIENT'S RESPIRATORY TRACT**
SYSTEM ZUR ULTRASCHALLDARSTELLUNG EINES ORGANS IM KÖRPER EINES PATIENTEN DURCH EINEN TEIL DER ATEMWEGE DES PATIENTEN
SYSTÈME D'IMAGERIE PAR ULTRASONS D'UN ORGANE DANS L ORGANISME D'UN PATIENT VIA UNE PARTIE DES VOIES RESPIRATOIRES DU PATIENT

(43) Date of publication of application: 29.12.2010
(73) Proprietor: Stroke2Prevent BV, 8022 AW Zwolle (NL)
(72) Inventor: NIERICH, Arno, NL-8051 CJ Hattem (NL)
(74) Representative: D'Halleweyn, Nele Veerle Trees Gertrudis
(86) International application number: PCT/EP2008/002209
(87) International publication number: WO 2009/115098

(56) References cited:
- WO-A-00/53098
- US-A- 5 190 046
- US-A1- 2003 208 119

## Description

The invention relates to a system according to the preamble of claim 1.

WO 00/53098 relates to an ultrasonic imaging method that is known as transesophageal echocardiography (TEE). TEE has become a widely used imaging technique for evaluating cardiac structure, function, and valvular anatomy. TEE has also provided a new perspective on the thoracic aorta, and there is growing evidence that the technique contributes valuable and sometimes unique information about aortic structure and pathology.

TEE involves introducing an echo probe into the patient's esophagus and transmitting ultrasound waves across the thorax in the direction of the heart and aorta. However, visualization of the ascending aorta by internal TEE is limited by an air structure, i.e. the trachea and main left and right bronchi. This is due to an important physical limitation of ultrasound: absorption of ultrasound waves. This absorption is dependent of the medium and expressed in terms of the "half power distance": the distance in which half of the ultrasound energy will be absorbed. For water this is 360 cm, bone 0,2 cm and for air 0,06 cm. This means that in practice ultrasound waves will not travel through bone or air.

Unfortunately, by the anatomical location of the aorta ascendens and the upper part of the main vascular side branches, it is difficult to view this area by TEE because the view is obstructed by the trachea. The trachea is located between the esophagus and the vascular tree, so all echoes are reflected by the trachea, which is filled with air.

In order to solve this problem, WO 00/53098 proposes the use of a balloon that may be arranged in the trachea or in one of the bronchi and that may be filled with an ultrasonic transmission fluid, e.g. water or a saline solution in minor concentrations. Obviously, this can only be done during operative surgery, when the patient is mechanically ventilated or on cardiopulmonary bypass, since in order to be effective the balloon has to completely fill and block the trachea or bronchus.

A problem which arises when trying to introduce the balloon in the left bronchus, which is the position of choice when visualizing the aorta ascendens, is that the flexible catheter carrying the balloon is hard to manipulate. Therefore, positioning the distal end of this flexible catheter in front of the left bronchus, so that the balloon may be lowered into that bronchus, is often a matter of trial and error. Since this positioning has to be performed during operative surgery, when timing is often critical, there is a clear need for an improvement.

The invention relates to a system for ultrasonic imaging of an organ in a patient' s body through a part of the patient's respiratory tract according to the preamble of claim 1. A prior art imaging system of this type is disclosed in WO 00/53098 and US 5 190 046.

The ultrasonic imaging system of the present invention is distinguished from these prior art systems by the features of the characterizing portion of claim 1.

The ultrasonic imaging system includes a short stylet, which is arranged between the inflatable member and a distal end of the catheter. Since this stylet does not extend across the inflatable member, it will not interfere with the imaging.

In a further development of this embodiment, the catheter may have a main lumen for filling the inflatable member with the ultrasonic transmission fluid, the stylet being arranged in this main lumen. In this way no structural modification of the catheter is required.

Alternatively, the catheter may have a main lumen for filling the inflatable member with the ultrasonic transmission fluid and an additional lumen for accommodating the stylet.

In yet another embodiment of the ultrasonic imaging system the catheter may have a main lumen for filling the inflatable member with the ultrasonic transmission fluid and the stylet may be fixedly arranged in a peripheral wall surrounding the main lumen.

The distal end of the catheter preferably includes a tip that is shaped to facilitate positioning in the patient' s left main bronchus and the stylet is adhesively fixed to the catheter tip. In this way the stylet and tip cooperate to allow the inflatable member to be optimally positioned.

The invention will now be illustrated by way of some exemplary embodiments, reference being made to the annexed drawings, in which corresponding parts are identified by reference numerals increased by 100, and in which:
Fig. 1 is a partly sectional view of a patient's upper body showing the ultrasonic imaging system of the invention during visualization of an organ,
Fig. 2 is an overview of a first embodiment of an ultrasonic imaging system not forming part of the invention, including a full length stylet, showing the inflatable member filled with transmission fluid,
Fig. 3 is a detailed, enlarged scale view of the encircled area III in Fig. 2,
Fig. 4 is a schematic view on an exaggerated scale of the distal end of the flexible catheter according to the first embodiment,
Fig. 5 is a view corresponding with Fig. 4, but showing a second embodiment not forming part of the invention,
Fig. 6 is a view corresponding with Figs. 4 and 5 and showing a third embodiment not forming part of the invention,
Figs. 7A to 7C are cross-sectional views of the catheter, showing the various possibilities for accommodating the full length stylet,
Fig. 8 is a detailed, enlarged scale view of the distal end of the catheter in a fourth embodiment, an embodiment of the invention, including a short stylet,
Fig. 9 is an overview of a fifth embodiment of an ultrasonic imaging system not forming part of the invention, including a pull wire,
Fig. 10 is a detailed, enlarged scale view of the encircled area X in Fig. 9,
Fig. 11 is a detailed, enlarged scale view of the distal end of the catheter in the fifth embodiment, and
Figs. 12A and 12B are cross-sectional views of the catheter, showing the various possibilities for accommodating the pull wire.

A method for ultrasonic imaging of an organ in a patient's body 1, in particular the heart or the aorta 2, through a part of the patient's respiratory tract 3, comprises the following steps.

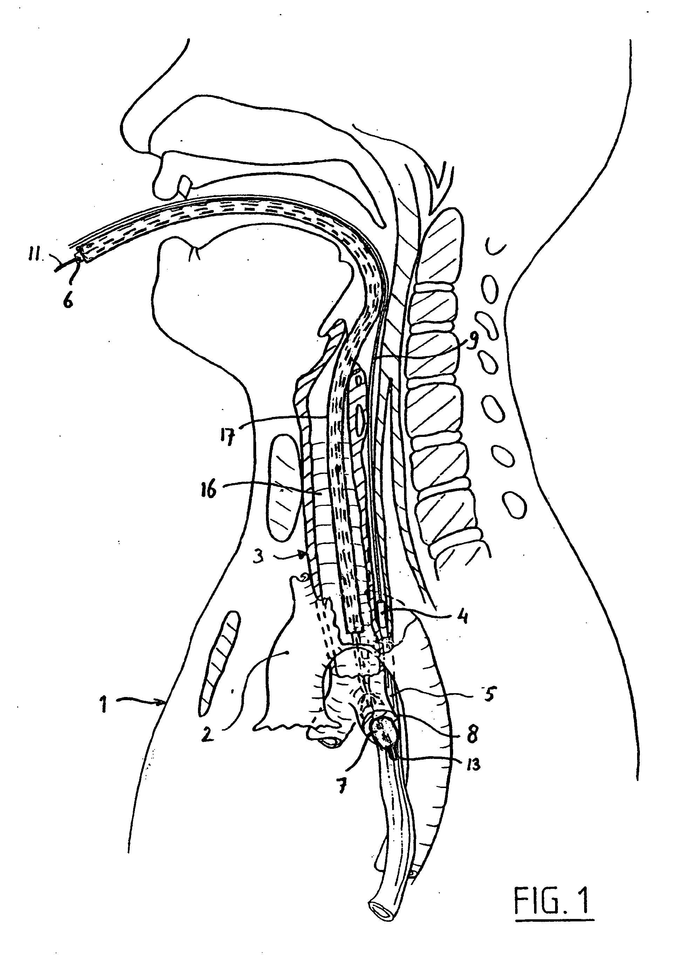

First an ultrasonic imaging device 4, for instance an echo probe, is arranged in or on the patient's body 1. In the shown embodiment, the echo probe 4, which is carried on a flexible catheter 9, is introduced into the patient's esophagus 5 (fig. 1). Then another flexible catheter 6 carrying an inflatable member 7 is introduced into the respiratory tract 3. The inflatable member 7 is positioned at a predetermined location in the respiratory tract 3. When the organ to be imaged is the ascending aorta 2, the predetermined position will be in the top part of the left bronchus 8.

In actual practice, the flexible catheter 6 carrying the inflatable member 7 will be guided through the patient's trachea 16 by first introducing an endotracheal tube 17 into the trachea 16. This tube 17 is somewhat stiffer than the catheter 6 and therefore easier to control. The catheter 6 is then inserted in the endotracheal tube 17. After leaving the endotracheal tube 17 the distal end 13 of the catheter 6 and the inflatable member 7 are guided into the left bronchus 8.

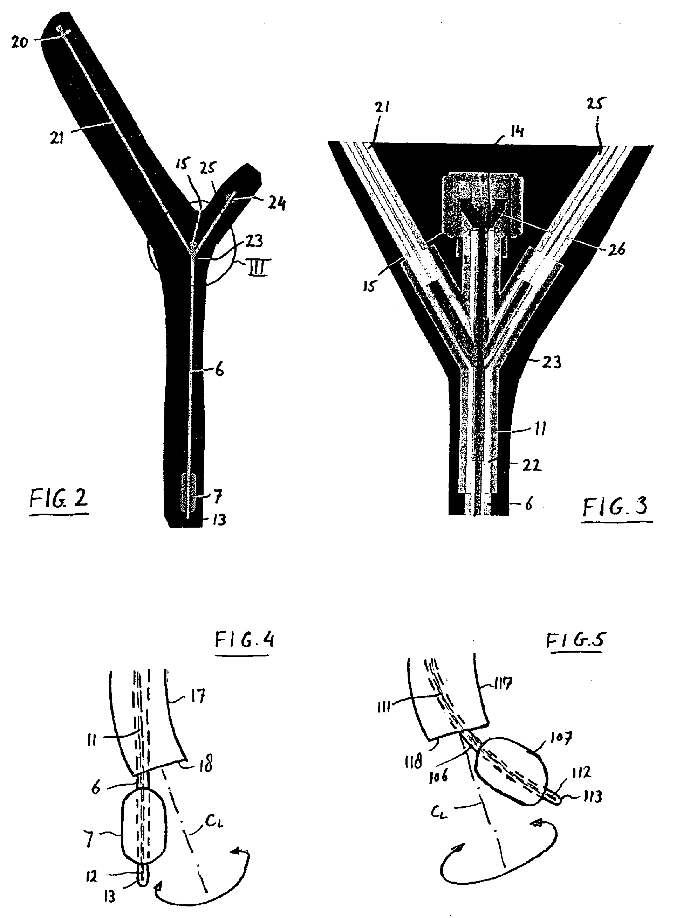

After the inflatable member 7 has been positioned, it is filled with an ultrasonic transmission fluid F through the flexible catheter 6. The fluid F is injected into the catheter 6 by means of a syringe (not shown), which is connected to a fill connector 20 at the end of a fill line 21 (fig. 2) . This fill line 21 in turn is connected to a proximal end 22 of the catheter 6 through a trident connector 23 (fig. 3). The degree of filling of the inflatable member 7 may be visually determined by monitoring a pilot balloon 24, which is arranged at the end of a pilot line 25. This pilot line 25 is also connected to the catheter 6 through the trident connector 23.

When the degree of inflation of the pilot balloon 24 indicates that the inflatable member 7 has been filled to such an extent that it completely covers the entire cross-sectional area of the left bronchus 8, so that no air is present between the echo probe 4 and the organ 2 to be imaged, the echo probe 4 is activated. Ultrasonic waves are then transmitted from the echo probe 4 through the transmission fluid F in the inflatable member 7 to the ascending aorta 2. Reflections from the aorta 2 are received at the echo probe 4 and transmitted through a line running through the catheter 9 to a processing and display apparatus, which does not form part of the present invention and is not shown here.

Due to the presence of the inflatable member 7 that is filled with the transmission fluid F, e.g. water or a saline solution in minor concentrations, the ultrasonic waves can travel pass the respiratory tract 3 with virtually no absorption. Consequently, very good ultrasound images of the aorta 2 may be obtained. Obviously, this can only be done during operative surgery, when the patient is mechanically ventilated or on cardiopulmonary bypass, since in order to be effective the inflatable member 7 has to completely fill and block the left bronchus 8.

In order to properly visualize the aorta it is important that the inflatable member 7 be positioned in precisely the right location. In accordance with the present invention the inflatable member 7 is positioned in the respiratory tract by manipulating guide means that are attached to or integrated with the flexible catheter 6.

In a first embodiment not forming part of the invention these guide means are passive and include a stylet 11 that extends over the entire length of the flexible catheter 6. A distal end 12 of the stylet 11 extends beyond the inflatable member 7 to a distal end 13 of the catheter 6. A proximal end 14 of the stylet 11 protrudes from the proximal end 22 of the catheter 6 outside the patient's body 1 and extends into the center prong of the trident connector 23. This center prong is closed by a cap 15 carrying a valve member 26, the function of which will be described below. This arrangement allows the inflatable member 7 to be swiftly and accurately positioned in the respiratory tract 3, since the presence of the stylet 11 adds stiffness to the flexible catheter 6, thus improving directional control and predictability of the movement.

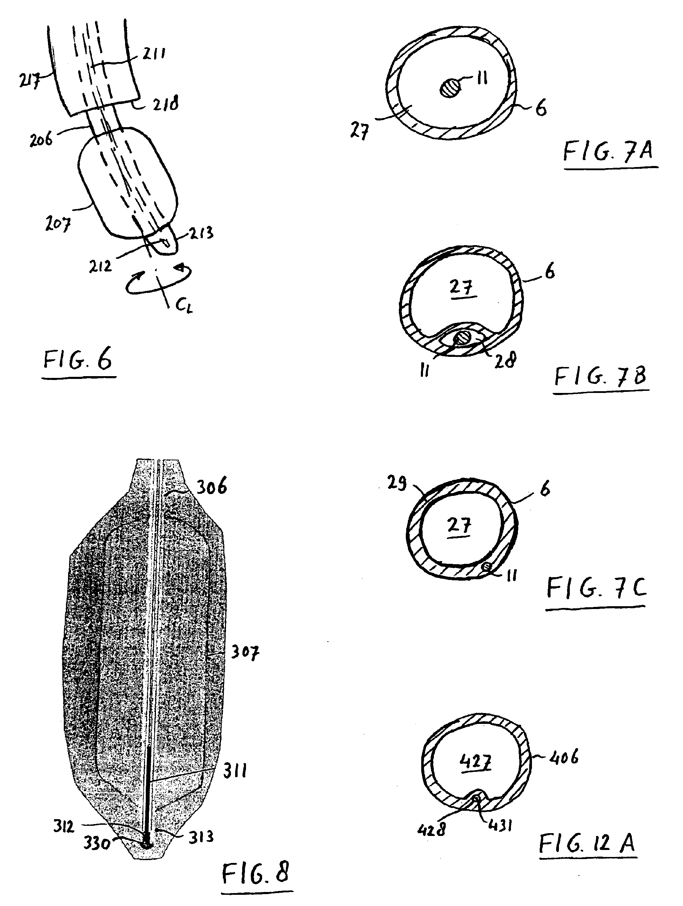

There are various possibilities for accommodating the full length stylet 11 in the catheter 6. In a first variant, which is structurally simple, the catheter 6 has a main lumen 27 for filling the inflatable member 7 with the ultrasonic transmission fluid F and the stylet 11 is arranged in the main lumen 27 (fig. 7A). Alternatively, the catheter 6 may have a main lumen 27 for the ultrasonic transmission fluid F and an additional lumen 28 for accommodating the stylet 11 (fig. 7B). In this way the stylet 11 does not interfere with the fluid supply function of the catheter 6.

In order to prevent the full length stylet 11 from obstructing part of the area to be imaged from view, it has to be at least partially retracted before the ultrasonic waves are transmitted from the imaging device 4. To that end the stylet 11 may be slidably arranged in either the main lumen 27 or the additional lumen 28. Although for proper imaging it would be sufficient to withdraw the stylet 11 only so far that its distal end 12 is on the proximal side of the inflatable member I1 in actual practice the stylet 11 will be completely withdrawn from the catheter 6, since it has served its purpose when the inflatable member 7 has been properly positioned. To allow the stylet 11 to be retracted after the inflatable member 7 has been filled with the transmission fluid F, without the risk of fluid F leaking from the system, the proximal end 14 of the stylet 11 protrudes from the catheter 6 through a valve member 26. In the illustrated embodiment this valve member 26 is a one-way valve that is arranged in the center prong of the trident connector 23.

Alternatively, the stylet 11 may be fixedly arranged in a peripheral wall 29 surrounding the main lumen 27 of the catheter 6 (fig. 7C). In this case a very thin stylet HA is used, which does not adversely affect the ultrasonic imaging in any substantial way, and which therefore does not have to be retracted. This thin stylet HA may be fixedly arranged in the catheter wall 29.

There are various possibilities for the stylet 11 and the catheter 6 carrying the inflatable member 7 to be guided to the predetermined position in e.g. the left bronchus 8.

In the illustrated embodiment the stylet 11 is made from a resilient material and is substantially straight. Consequently, the stylet 11 will always try to assume its original straight shape. When the flexible catheter 6 and the stylet 11 are guided through the endotracheal tube 17, which itself follows the curvature of the trachea 16, they will have to follow the curvature of the tube 17 as well. However, after leaving the distal end 18 of the endotracheal tube 17 the stylet 11 will return to its straight shape due to its resiliency. The part of the stylet 11 and the catheter 6 protruding from the distal end 18 of the tube 17 will therefore enclose an angle with an imaginary extension of the centerline CL of the tube 17. Consequently, rotating the proximal end 14 of the stylet 11 will lead to the distal end 12 describing a circular motion, which facilitates positioning of the distal end 13 of the catheter 6 at the entrance of the left bronchus 8 (fig. 4).

In an alternative embodiment not forming part of the invention the stylet 111 is made from a resilient material and is preformed to conform substantially to the intended path of the catheter 106 through the respiratory tract 3. Preforming the stylet 111 may advantageously be done before it is attached to or integrated with the flexible catheter 106. In this way the catheter 106 assumes the shape of the stylet 111. When the flexible catheter 106 and the stylet 111 are guided through the endotracheal tube 117, which has less curvature than the stylet 111, they will be straightened somewhat. However, after leaving the distal end 118 of the endotracheal tube 117 the stylet 111 will return to its curved shape due to its resiliency, again extending under an angle with respect to the extension of the centerline CL of the tube 117. Therefore, also in this embodiment rotating the proximal end 114 of the stylet 111 will result in a circular motion of its distal end 112, thus allowing the distal end 113 of the catheter 106 to be positioned at the entrance of the left bronchus 8 (fig. 5).

In a third embodiment not forming part of the invention the stylet 211 is made from a deformable material. In this way, the stylet 211 will conform to the respiratory tract 3, or to the endotracheal tube 217, when the flexible catheter 206 including this stylet 211 passes through the trachea 16. After leaving the tube 217 the material of the stylet 211 will maintain the curved shape into which it was forced. Consequently, the distal end 213 of the catheter 206 will form a continuation of the curvature of the tube 217, so that it can again easily be guided to its predetermined position at the entrance of the left bronchus 8 (fig. 6) by manipulating the proximal end of the stylet 211.

In yet another embodiment, an embodiment of the invention, the ultrasonic imaging system may include a short stylet 311, which may be arranged between the inflatable member 307 and the distal end 313 of the catheter 306 (fig. 8), rather than a full length stylet. Since this short stylet 311 does not extend across the inflatable member 307, it will not interfere with the imaging and there is no need to retract it. The distal end 313 of the catheter 306 includes a tip 330 that is shaped to facilitate positioning in the patient's left main bronchus 8 and the stylet 311 is adhesively fixed to the catheter tip 330. In this way the stylet 311 and tip 330 cooperate to allow the inflatable member 307 to be optimally positioned.

Since the stylet 311 does not extend beyond the distal end of the inflatable member 307, this embodiment will not have any part extending outside the patient's body 1. Positioning of the catheter 306 and the inflatable member 307 is done by manipulating the proximal end of the catheter 306. And since this stylet 311 does not have to be retracted from the catheter 306, there is no need for a special valve means.

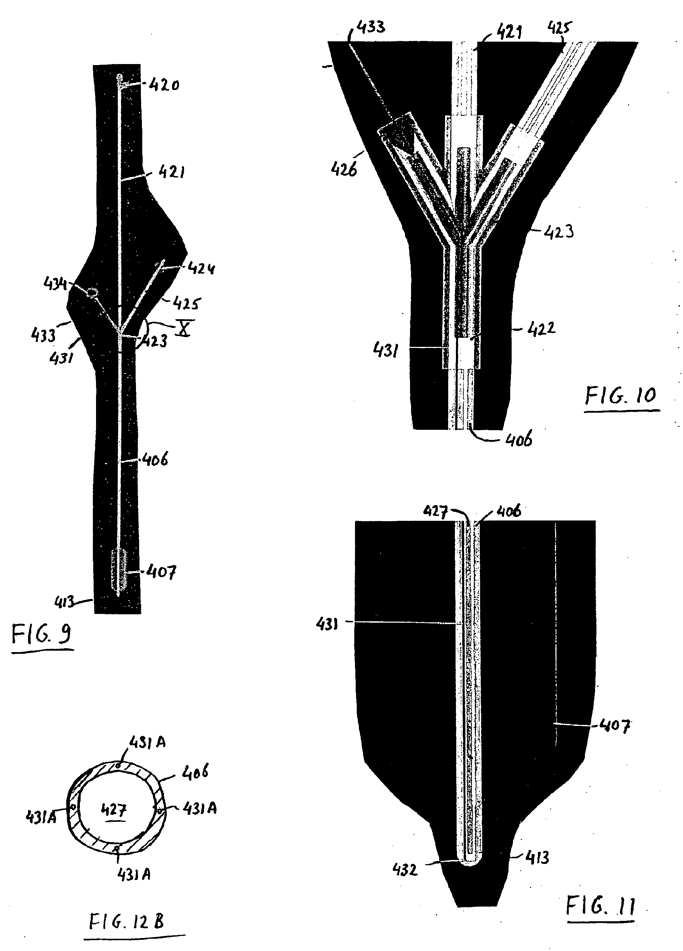

In another main embodiment of an ultrasonic imaging system not forming part of the invention the guide means 410 comprise a wire 431 rather than a stylet. A distal end 432 of the wire 431 is eccentrically connected to the flexible catheter 406 and a proximal end 433 of the wire 431 is connected to a pulling member 434 arranged outside the patient's body 1 (fig. 9). The wire 431, which is very thin, provides excellent guidance of the catheter 406 with minimum obstruction of the image. The inflatable member 407 is positioned in the respiratory tract 3 by manipulating the pulling member 434. By pulling on the wire 431, its effective length within the catheter 406 will decrease. Since the wire 431 is eccentrically attached to the catheter 406, shortening of the wire 431 will lead to the catheter 406 assuming a curved shape, at least in the vicinity of the point where the wire 431 is attached. In the illustrated embodiment this attachment point is located near the distal end 413 of the catheter 406. This location allows optimum control of the catheter 406.

In this embodiment the inflatable member 407 is again filled by means of a syringe which may be connected to a fill connector 420 at the end of a fill line 421. This fill line 421 is again connected to the catheter 406 through a trident connector 423, in this case through the center prong thereof. Also connected to the trident connector 423 is a pilot line 425 carrying a pilot balloon 424. Finally, the proximal end of the wire 431 is guided through the third prong of the trident connector 423. In order to prevent fluid leakage, the proximal end 433 of the pull wire 431 protrudes from this third prong through a valve member, in particular a one-way valve 426 (fig. 10).

There are various possibilities for accommodating the pull wire 431 in the catheter 406. The pull wire 431 may be accommodated in the main lumen 427 of the catheter 406 (fig. 11) . This has the advantage of that the use of the guide means 410 entails a minimum of structural modifications. Alternatively, the catheter 406 may have an additional lumen 428 for accommodating the pull wire 431, besides the main lumen 427 for filling the inflatable member 407 with the ultrasonic transmission fluid F (fig. 12A). In this way interference between the pull wire 431 and the transmission fluid F is again minimized.

Instead of just a single pull wire, the ultrasonic imaging system may comprise a plurality of pull wires 431A (fig. 12B), which are spaced in the peripheral direction of the catheter 406. This allows the catheter 406 to be controlled in different directions by manipulating the various pull wires 431. Additionally or alternatively, the distal ends of the various wires 431A may also be spaced in lengthwise direction of the catheter 406 to further improve controllability of the catheter 406.

Thus, the invention provides a system with which an inflatable member that is to be filled with an ultrasound transmission fluid may be swiftly and accurately brought into a predetermined position within the respiratory tract of a patient. This in turn allows certain parts of the circulatory system, in particular the heart or aorta, to be visualized through the respiratory tract, using an imaging device that is arranged in the patient's esophagus, thus providing valuable information during operative surgery.

Although the invention has been described by means of a number of examples, it will be clear to the skilled person that many variations or modifications may be envisaged within the scope of the appended claims.

## Claims

1. A system for ultrasonic imaging of an organ in a patient's body through a part of the patient's respiratory tract, comprising:
- an ultrasonic imaging device (4) to be arranged in or on the patient's body,
- a flexible catheter (306) carrying at least one inflatable member (307) to be arranged in the respiratory tract,
- means for positioning the inflatable member at a predetermined location in the respiratory tract (3), and
- means for filling the inflatable member with an ultrasonic transmission fluid through the flexible catheter,
**characterized in that** the positioning means comprise a short stylet (311) that is attached to or integrated with the flexible catheter (306), said stylet being arranged between the inflatable member (307) and a distal end (313) of the catheter such that said stylet (311) does not extend across the inflatable member (307).

2. The ultrasonic imaging system of claim 1, wherein the stylet (311) is arranged in the catheter and has a distal end (313) which extends beyond the inflatable member (307).

3. The ultrasonic imaging system of claim 2, wherein the catheter has a main lumen (27) for filling the inflatable member with the ultrasonic transmission fluid and wherein the stylet (11) is arranged in the main lumen.

4. The ultrasonic imaging system of claim 2, wherein the catheter has a main lumen (27) for filling the inflatable member with the ultrasonic transmission fluid and an additional lumen (28) for accommodating the stylet (11).

5. The ultrasonic imaging system of claim 2, wherein the catheter has a main lumen (27) for filling the inflatable member with the ultrasonic transmission fluid and wherein the stylet (11) is fixedly arranged in a peripheral wall (29) surrounding the main lumen.

6. The ultrasonic imaging system of any of claims 1 to 5, wherein the stylet (11) is made from a resilient material.

7. The ultrasonic imaging system of any of the claims 1 to 6, wherein the stylet is substantially straight.

8. The ultrasonic imaging system of any of the claims 1 to 7, wherein the stylet is made from a deformable material.

9. The ultrasonic imaging system of any of the claims 1 to 8, wherein the distal end of the catheter includes a tip that is shaped to facilitate positioning in the patient's left main bronchus and the stylet is adhesively fixed to the catheter tip.

## Patentansprüche

1. System zur Ultraschalldarstellung eines Organs in einem Körper eines Patienten durch einen Teil der Atemwege des Patienten, welches umfasst:
- eine Ultraschalldarstellungsvorrichtung (4), anzuordnen in oder an dem Körper des Patienten,
- einen flexiblen Katheter (306), der wenigstens ein aufblasbares Element (307) trägt, anzuordnen in den Atemwegen,
- Mittel zum Positionieren des aufblasbaren Elements an einer vorgegebenen Stelle in den Atemwegen (3), und
- Mittel zum Füllen des aufblasbaren Elements mit einem Ultraschalltransmissionsfluid durch den flexiblen Katheter,
**dadurch gekennzeichnet, dass** das Positionierungsmittel einen kurzen Führungsstab (311) umfasst, der angefügt ist an oder integriert ist mit dem flexiblen Katheter (306), wobei der Führungsstab zwischen dem aufblasbaren Element (307) und einem distalen Ende (313) des Katheters angeordnet ist, so dass der Führungsstab (311) sich nicht über das aufblasbare Element (307) erstreckt.

2. Ultraschalldarstellungssystem nach Anspruch 1, wobei der Führungsstab (311) in dem Katheter angeordnet ist und ein distales Ende (313) aufweist, das sich über das aufblasbare Element (307) hinaus erstreckt.

3. Ultraschalldarstellungssystem nach Anspruch 2, wobei der Katheter ein Hauptlumen (27) zum Füllen des aufblasbaren Elements mit dem Ultraschalltransmissionsfluid aufweist, und wobei der Führungsstab (11) in dem Hauptlumen angeordnet ist.

4. Ultraschalldarstellungssystem nach Anspruch 2, wobei der Katheter ein Hauptlumen (27) zum Füllen des aufblasbaren Elements mit dem Ultraschalltransmissionsfluid und ein Zusatzlumen (28) zur Aufnahme des Führungsstabs (11) aufweist.

5. Ultraschalldarstellungssystem nach Anspruch 2, wobei der Katheter ein Hauptlumen (27) zum Füllen des aufblasbaren Elements mit dem Ultraschalltransmissionsfluid aufweist, und wobei der Führungsstab (11) fest in einer Umfangswand (29), die das Hauptlumen umgibt, angeordnet ist.

6. Ultraschalldarstellungsystem nach einem der Ansprüche 1 bis 5, wobei der Führungsstab (11) aus einem elastischen Material hergestellt ist.

7. Ultraschalldarstellungssystem nach einem der Ansprüche 1 bis 6, wobei der Führungsstab im wesentlichen gerade ist.

8. Ultraschalldarstellungssystem nach einem der Ansprüche 1 bis 7, wobei der Führungsstab aus einem deformierbaren Material hergestellt ist.

9. Ultraschalldarstellungssystem nach einem der Ansprüche 1 bis 8, wobei das distale Ende des Katheters eine Spitze einschließt, die so geformt ist, um ein Positionieren im linken Stammbronchus des Patienten zu erleichtern, und wobei der Führungsstab anhaftend an der Katheterspitze befestigt ist

## Revendications

1. Dispositif d'imagerie à ultrasons d'un organe dans un corps de patient à travers une partie des voies respiratoires du patient, comprenant :
- un dispositif d'imagerie à ultrasons (4) destiné à être agencé dans ou sur le corps du patient,
- un cathéter souple (306) comportant au moins un élément gonflable (307) destiné à être agencé dans les voies respiratoires,
- des moyens destinés à positionner l'élément gonflable à un emplacement prédéterminé dans les voies respiratoires (3), et
- des moyens destinés à remplir l'élément gonflable avec un fluide de transmission d'ultrasons à travers le cathéter souple,
**caractérisé en ce que** le moyen de positionnement comprend un stylet court (311) qui est fixé sur le cathéter souple (306) ou intégré à ce dernier, ledit stylet étant agencé entre l'élément gonflable (307) et une extrémité distale (313) du cathéter de telle sorte que ledit stylet (311) ne s'étende pas à travers l'élément gonflable (307).

2. Dispositif d'imagerie par ultrasons selon la revendication 1,
dans lequel le stylet (311) est agencé dans le cathéter et présente une extrémité distale (313) qui s'étend au-delà de l'élément gonflable (307).

3. Dispositif d'imagerie par ultrasons selon la revendication 2, dans lequel le cathéter comporte une lumière principale (27) destinée à remplir l'élément gonflable avec le fluide de transmission d'ultrasons et dans lequel le stylet (11) est agencé dans la lumière principale.

4. Dispositif d'imagerie par ultrasons selon la revendication 2, dans lequel le cathéter comporte une lumière principale (27) destinée à remplir l'élément gonflable avec un fluide de transmission d'ultrasons et une lumière supplémentaire (28) destinée à recevoir le stylet (11).

5. Dispositif d'imagerie par ultrasons selon la revendication 2, dans lequel le cathéter comporte une lumière principale (27) destinée à remplir l'élément gonflable avec un fluide de transmission d'ultrasons et dans lequel le stylet (11) est agencé à demeure dans une paroi périphérique (29) entourant la lumière principale.

6. Dispositif d'imagerie par ultrasons selon l'une quelconque des revendications 1 à 5, dans lequel le stylet (11) est réalisé en un matériau souple.

7. Dispositif d'imagerie par ultrasons selon l'une quelconque des revendications 1 à 6, dans lequel le stylet est sensiblement rectiligne.

8. Dispositif d'imagerie par ultrasons selon l'une quelconque des revendications 1 à 7, dans lequel le stylet est réalisé en un matériau déformable.

9. Dispositif d'imagerie par ultrasons selon l'une quelconque des revendications 1 à 8, dans lequel l'extrémité distale du cathéter comporte un embout qui est façonné de manière à faciliter le positionnement dans la bronche principale gauche du patient et le stylet est fixé par adhésif sur l'embout du cathéter.
